# EUROPÄISCHE PATENTSCHRIFT

(11) **EP 2 621 430 B1**
(45) Veröffentlichungstag und Bekanntmachung des Hinweises auf die Patenterteilung: **05.11.2014**
(21) Anmeldenummer: 11760403.3
(22) Anmeldetag: 08.09.2011
(51) Int. Cl.: A61F 13/15, B65B 25/14, B65B 61/02, B65B 65/00, B65B 9/06

(54) **VERFAHREN ZUM VERPACKEN VON PRODUKTEN UND VERPACKUNGSANLAGE ZUR DURCHFÜHRUNG DES VERFAHRENS**
METHOD FOR PACKAGING PRODUCTS AND PACKAGING PLANT FOR CARRYING OUT THE METHOD
PROCEDURE D'EMBALLAGE DE PRODUITS ET INSTALLATION POUR LA REALISATION DE LA PROCEDURE

(30) Priorität: 27.09.2010 DE 102010046561
(43) Veröffentlichungstag der Anmeldung: 07.08.2013
(73) Patentinhaber: Winkler + Dünnebier GmbH, 56564 Neuwied (DE)
(72) Erfinder: SEGER, Reiner, 56566 Neuwied (DE); WALD, Ulrich, 56170 Bendorf-Sayn (DE); SCHNELL, Marc, 56218 Mühlheim-Kärlich (DE); LUGOJA, Lucian-Gabriel, 67596 Dittelsheim-Hessloch (DE); RINKE, Andreas, 23843 Bad Oldensee (DE)
(74) Vertreter: Tergau & Walkenhorst
(86) Internationale Anmeldenummer: PCT/EP2011/004570
(87) Internationale Veröffentlichungsnummer: WO 2012/041444

(56) Entgegenhaltungen:
- US-B1- 6 688 077

## Beschreibung

Die Erfindung betrifft ein Verfahren zum Verpacken von Produkten, insbesondere von Hygieneprodukten, in Verpackungsbeutel. Sie bezieht sich weiter auf eine Verpackungsanlage zum Verpacken von Produkten in Verpackungsbeutel, insbesondere zur Durchführung des Verfahrens.

Verpackungsanlagen allgemein sind z.B. aus US6688077B bekannt. Hygieneprodukte wie beispielsweise Papiertaschentücher, Slipeinlagen, Damenbinden, Windeln oder dergleichen werden üblicherweise einzeln oder in vorgegebenen Chargen, beispielsweise in der Art von Fünfer- oder Zehnerpacks, in Folie verpackt und in dieser verpackten Form verbrauchs- oder gebrauchsfertig angeboten. Die das Hygieneprodukt bzw. den Stapel der Hygieneprodukte umgebende Folie bildet dabei einen so genannten Verpackungsbeutel, der u.a. beispielsweise mit einer Perforation zum erleichterten öffnen und/oder mit Klebelaschen oder dergleichen zum Wiederverschließen ausgerüstet sein kann. Gleichermaßen können auch andere Produkte oder Produktchargen, wie beispielsweise Zigarettenpackungen oder dergleichen, mit einem derartigen Verpackungsbeutel in der Art einer Umverpackung versehen werden.

Zum Einbringen des Produkts, insbesondere des Hygieneprodukts oder des Stapels von Hygieneprodukten, in den Verpackungsbeutel kann es beispielsweise vorgesehen sein, dass geeignet zugeschnittene, zur Bildung des Verpackungsbeutels vorgesehene Folienstücke bereitgestellt werden, in die das Produkt eingelegt wird, wobei die Folie anschließend um dieses herumgeschlagen und an den Rändern verschweißt wird. Ein derartiges Konzept zum Verpacken von Hygieneprodukten in einen Verpackungsbeutel ist beispielsweise aus der DE 101 48 283 A1 bekannt, bei der ein so genanntes Zellenrad für den eigentlichen Verpackungsschritt vorgesehen ist. Dabei wird die zur Bildung des Verpackungsbeutels vorgesehene Folie zunächst in eine Kammer des Zellenrades eingelegt, wobei sodann ein Stapel von Hygieneprodukten ebenfalls in die Kammer des Zellenrades eingebracht wird. Anschließend werden die Seitenränder der Folie umlaufend um den Stapel von Hygieneprodukten herum in Überlappung gebracht und miteinander geeignet verschweißt.

Alternativ kann auch ein so genannter Folienschlauch-Verpacker zum Einsatz kommen, wie er beispielsweise aus der DE 10 2008 020 800 A1 bekannt ist. In einem derartigen System werden die zu verpackenden Hygieneprodukte bzw. Stapel von Hygieneprodukten in einer so genannten Schlauchbeutelmaschine in einen Folienschlauch positioniert, der dann zwischen den Produkten zusammengeschweißt und getrennt wird.

Gerade beim Verpacken von Hygieneprodukten, die in besonders großer Stückzahl hergestellt werden, wie beispielsweise Papiertaschentücher, Slipeinlagen, Damenbinden oder dergleichen, ist eine hohe Bearbeitungsgeschwindigkeit und Durchsatzrate beim Verpacken der Produkte wünschenswert. Aus diesem Grund werden die Verpackungssysteme üblicherweise in der Art von Durchlaufsystemen ausgelegt. Bei diesen werden die zu verpackenden Produkte aufeinander folgend, einzeln oder stapelweise entlang einer Transportrichtung weiterbefördert und während des Durchlaufs durch verschiedene, entlang der Transportrichtung angeordnete Systemkomponenten mit dem Verpackungsbeutel umgeben. Bei den bekannten Systemen, also beispielsweise bei dem beschriebenen zellenradbasierten Einschlagen der Hygieneprodukte in geeignet vorgeschnittene Folienstücke oder auch bei den genannten Folienschlauchsystemen, ist jedoch üblicherweise beim Verschweißen der Seitenbereiche des Verpackungsbeutels ein kurzfristiger Systemstillstand in Kauf zu nehmen, bei dem der Weitertransport der Produkte vorübergehend unterbrochen wird. Derartige, auch kurzfristige Unterbrechungen des Weitertransports, die insbesondere für die Schweißschritte notwendig werden, reduzieren dabei in unerwünschter Weise die Durchsatz- und Produktionsrate des Systems.

Der Erfindung liegt daher die Aufgabe zu Grunde, ein Verfahren zum Verpacken von Produkten, insbesondere von Hygieneprodukten, in einen Verpackungsbeutel anzugeben, mit dem besonders hohe Durchsatz- und Produktionsraten erreichbar sind und somit eine besonders hohe Produktionsgeschwindigkeit bei hoher Zuverlässigkeit gewährleistet ist. Des Weiteren soll eine zur Durchführung des Verfahrens besonders geeignete Verpackungsanlage angegeben werden.

Bezüglich des Verfahrens wird diese Aufgabe erfindungsgemäß gelöst, indem die zu verpackenden Produkte in einen Beutelfolienschlauch eingeschlagen werden und dieser anschließend in einer Transportrichtung durch eine erste Schweißstation und durch eine zweite Schweißstation geführt wird, wobei der mit den Produkten versehene Beutelfolienschlauch in einem ersten Schweißschritt seitlich verschweißt wird, bevor in einem zweiten Schweißschritt seine Stirnseiten verschweißt werden, und wobei zwischen den Schweißschritten der mit den Produkten versehene Beutelfolienschlauch relativ zur Transportrichtung um einen dem von zwei aneinanderstoßenden Produktseiten aufgespannten Winkel entsprechenden Verdrehwinkel gedreht wird.

Die Erfindung geht dabei von der Überlegung aus, dass zur Erreichung besonders hoher Durchsatz- und Produktionsraten ein möglichst kontinuierlicher und unterbrechungsfreier Durchlauf der Produkte oder Produktstapel durch das Verpackungssystem angestrebt werden sollte. Um dabei Systemstillstände oder auch kurzfristige Unterbrechungen des Weitertransports der Produkte oder Produktstapel weitestgehend zu verhindern, sollten sämtliche Bearbeitungsschritte beim Einbringen der Produkte oder Produktstapel in die jeweiligen Verpackungsbeutel konsequent für einen Durchlaufbetrieb ausgelegt sein. Um dies gerade auch bei den Schweißschritten, bei denen im Randbereich des jeweiligen Verpackungsbeutels die überlappenden Randstücke miteinander verschweißt werden, zu gewährleisten, sollten die Schweißschritte konsequent für einen Zugriff auf das durchlaufende Produkt von der Seite her ausgelegt sein. Dazu sollten die jeweiligen Schweißspiegel, die den erforderlichen Wärmeeintrag in das Folieenmaterial sicherstellen, der schließlich zum Verschweißen der überlappenden Folienteile miteinander führt, jeweils seitlich zur Transportrichtung der Produkte oder Produktstapel angeordnet sein, so dass die Produkte oder Produktstapel in Transportrichtung gesehen jeweils zwischen den Schweißspiegeln oder Heizelementen hindurchgeführt werden können.

Um dies für sämtliche miteinander zu verschweißende Seitenbereiche des Verpackungsbeutels, üblicherweise zunächst eine der Längsseiten und anschließend die Stirnseiten, zu gewährleisten, sollte der Schweißvorgang zweistufig vorgenommen werden. Dabei kann in einem ersten Schweißschritt unter Zugriff von der Seite her beispielsweise zunächst die offene Längsseite des Verpackungsbeutels verschweißt werden. Anschließend ist eine Drehung des in den Beutelfolienschlauch eingeschlagenen Produkts oder Produktstapels relativ zur Transportrichtung derart vorgesehen, dass die zu diesem Zeitpunkt noch nicht verschweißten Produktseiten, bezogen auf die Transportrichtung, nach der Drehung nunmehr seitlich ausgerichtet sind. Somit kann im nachfolgenden zweiten Schweißschritt auch die Verschweißung der Stirnseiten von der Seite her vorgenommen werden

Die Verdrehung sollte dabei entsprechend der Grundform oder Grundfläche des Produkts oder Produktstapels geeignet erfolgen. Beispielsweise könnte das Produkt dabei eine im Wesentlichen dreieckige Grundform haben, so dass die Verdrehung um einen der Dreieckswinkel erfolgt. Für Produkte mit im Wesentlichen rechteckiger Grundfläche wie beispielsweise Zigarettenschachteln, Taschentücher oder dergleichen wird zweckmäßigerweise ein Verdrehwinkel von 90° gewählt. Das Verdrehen kann dabei erfolgen, indem die Transportrichtung beibehalten und das Produkt gedreht wird, oder auch, indem die Orientierung des Produkts beibehalten und die Transportrichtung geeignet verändert wird.

Die zur Bildung des Verpackungsbeutels vorgesehene Beutelfolie kann grundsätzlich bereits vor der Einbringung des Produkts oder Produktstapels geeignet auf die erforderlichen Maße zugeschnitten und entsprechend bereit gestellt werden. Um aber auf besonders einfache Weise die erwünschten hohen Durchsatz- oder Produktionsraten weiter zu begünstigen, werden die Produkte oder Produktstapel vorteilhafterweise in einen Folienschlauch eingeschlagen, wobei eine Abfolge einzelner Produkte oder Produktstapel in die Beutelfolie eingebracht und diese anschließend in einem ersten Schweißschritt entlang ihrer Längsrichtung zu einem Folienschlauch verschweißt wird. Um auch bei einer derartigen Ausgestaltung die vorgesehene Verdrehung der in den Folienschlauch eingeschlagenen Produkte oder Produktstapel um den gewünschten Winkel relativ zur Transportrichtung zu ermöglichen, wird der mit den Produkten versehene Beutelfolienschlauch vorteilhafterweise nach dem ersten Schweißschritt in jeweils ein oder einige Produkte oder Produktstapel beinhaltende Verpackungsbeutel vereinzelt.

Im Hinblick auf eine besonders weitgehende Steigung der erreichbaren Produktionsoder Durchsatzraten ist in besonders vorteilhafter Weiterbildung vorgesehen, den zweiten Schweißschritt, also die Stirnschweißung der bereits in den Folienschlauch eingeschlagenen Produkte oder Produktstapel, in der Art einer parallelen oder mehrkanaligen Verarbeitung in zwei oder mehr parallel geschalteten Weiterverarbeitungssträngen vorzunehmen. Dazu wird vorteilhafterweise der mit den Produkten oder Produktstapeln versehene Beutelfolienschlauch nach dem ersten Schweißschritt zunächst in Einheiten vereinzelt, die zwei oder gegebenenfalls drei oder mehr aufeinanderfolgende Produkte oder Produktstapel umfassen. Diese Einheiten werden vorteilhafterweise anschließend als Ganzes geeignet umgeleitet oder gedreht, so dass die gewünschte Verdrehung bezogen auf die Transportrichtung entsteht. Anschließend werden diese Einheiten vorteilhafterweise ihrerseits geeignet zertrennt, so dass einzelne jeweils in Folienschlauch eingeschlagene Produkte oder Produktstapel vorliegen. Diese können dann in der Art einer parallelen Weiterverarbeitung den nachfolgenden, jeweils mit einer separaten Schweißstation für die jeweilige Stirnschweißung versehenen Weiterverarbeitungssträngen zugeführt werden.

Gerade bei der Verpackung der Hygieneprodukte in vergleichsweise hohen Stückzahlen kann es wünschenswert oder sogar erforderlich sein, die Produkte oder Produktstapel innerhalb des Verpackungsbeutels relativ zu diesem präzise und in Einklang mit vorgegebenen Randbedingungen oder Erfordernissen, beispielsweise korrekt positioniert in Relation zu einer zur Bildung einer Zugriffsöffnung vorgesehenen Perforation oder in Relation zu einer äußeren Bedruckung, im Verpackungsbeutel zu positionieren. Um dies zuverlässig zu ermöglichen, werden die zu verpackenden Hygieneprodukte der zur Bildung des Beutelfolienschlauchs vorgesehenen Schlauchfolie vorteilhafterweise synchronisiert zu einer in der Schlauchfolie angebrachten Perforierung zugeführt.

Bezüglich der Verpackungsanlage zum Verpacken von Hygieneprodukten in Verpackungsbeutel wird die genannte Aufgabe gelöst mit einem Transportsystem, über das die zu verpackenden Hygieneprodukte von einer Einschlagstation zunächst einer ersten Schweißstation und von dieser aus einer zweiten Schweißstation zuführbar sind, und mit einer in Transportrichtung der Hygieneprodukte gesehen zwischen der ersten und der zweiten Schweißstation angeordneten Umlenkeinheit zur Drehung der mit den Hygieneprodukten versehenen Verpackungsbeutel relativ zur Transportrichtung um einen vorgesehenen Verdrehwinkel, vorzugsweise um 90°.

Vorteilhafterweise weist die Verpackungsanlage zudem eine in Transportrichtung gesehen nach der ersten Schweißstation angeordnete Vereinzelungseinheit für die Verpackungsbeutel auf.

Die mit der Erfindung erzielten Vorteile bestehen insbesondere darin, dass durch die vorgesehene Aufteilung bei der Verschweißung insbesondere des Randbereichs des Verpackungsbeutels in zwei Schweißschritte, zwischen denen eine Drehung des mit den Hygieneprodukten versehenen Verpackungsbeutels relativ zur Transportrichtung um den geeigneten Verdrehwinkel von insbesondere etwa 90° vorgesehen ist, die Verschweißung des Randbereichs des Verpackungsbeutels vollständig und ausschließlich von der Seite her und damit ohne Behinderung des Weitertransports der Produkte möglich ist. Somit kann das Verpacken der Hygieneprodukte in die jeweiligen Verpackungsbeutel vollständig in der Art eines Durchlaufbetriebs erfolgen, so dass unerwünschte, die Durchsatz- und Produktionsrate begrenzende Systemstillstände beim Verschweißen vermieden sind. Zudem kann durch die der Drehung nachgelagerte Weiterverarbeitung und insbesondere Stirnschweißung der eingeschlagenen Produkte oder Produktstapel in mehreren parallel geschalteten Weiterverarbeitungssträngen der Durchsatz besonders hoch eingestellt werden.

Ein Ausführungsbeispiel der Erfindung wird anhand einer Zeichnung näher erläutert. Darin zeigen:
- FIG. 1: eine Verpackungsanlage zum Verpacken von Hygieneprodukten,
- FIG. 2: schematisch und ausschnittsweise das Transportsystem der Verpackungsanlage nach FIG. 1, und
- FIG. 3: eine alternative Ausführungsform des Transportsystems nach FIG. 2.

Gleiche Teile sind in beiden Figuren mit denselben Bezugszeichen versehen.

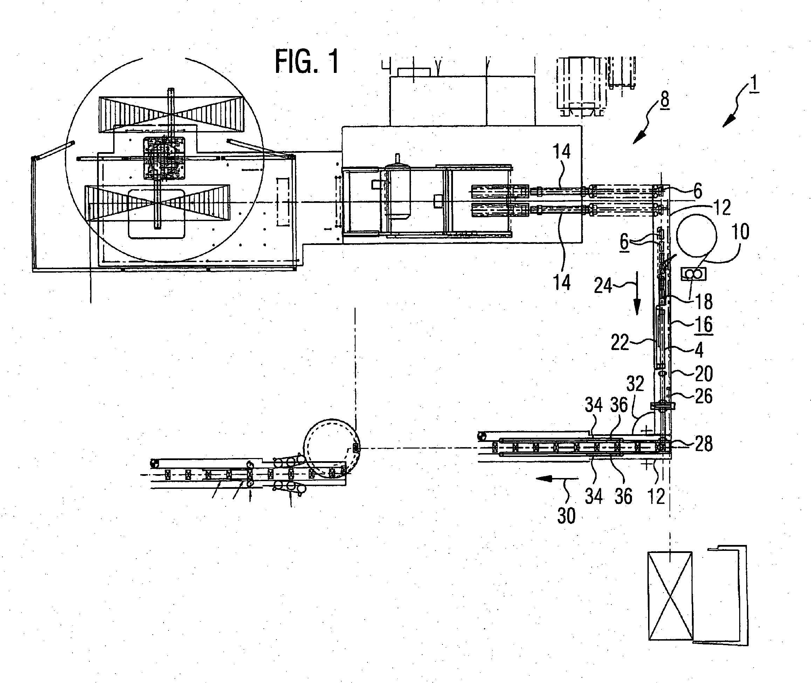

Die Verpackungsanlage 1 gemäß FIG. 1 ist zum Verpacken von Hygieneprodukten in einen Verpackungsbeutel vorgesehen. Im Ausführungsbeispiel sollen dabei als Hygieneprodukte Papiertaschentücher chargenweise, also als Stapel einzelner Taschentücher, mit einer Chargengröße von jeweils zehn Stück in jeweils einen Verpackungsbeutel eingebracht werden. Alternativ könnten selbstverständlich aber auch andere Chargengrößen, beispielsweise die Verpackung von drei oder fünf Papiertaschentüchern in einen Verpackungsbeutel, oder auch andere Hygieneprodukte wie beispielsweise Slipeinlagen, Damenbinden oder dergleichen, vorgesehen sein.

Die Verpackungsanlage 1 ist dabei als so genannter Folienschlauch-Verpacker ausgelegt, bei dem die zu verpackenden Papiertaschentücher chargen- oder stapelweise zunächst in einen Beutelfolienschlauch 4 eingeschlagen werden. Dazu umfasst die Verpackungsanlage 1 eine Zuführeinheit 8, über die die Papiertaschentücher in Form von Produktstapeln 6, jeweils umfassend zehn übereinander liegende Papiertaschentücher, einem Transportsystem 12 zugeführt werden. Die Zuführeinheit 8 ist dabei im Ausführungsbeispiel zweibahnig ausgeführt, d. h. in der Art einer parallelen Anordnung zweier Zuführschienen 14 werden die Produktstapel 6 parallel zueinander dem Transportsystem 12 zugeführt. Diese zweibahnige Anordnung ermöglicht insbesondere die Zusammenführung mehrerer Transportstränge für eine gemeinsame Weiterverarbeitung der Produktstapel 6. Damit sind - im Vergleich zur Bereitstellungsrate der Produktstapel 6 bei deren Herstellung - verdoppelte Durchsatzrate beim Verpacken und bei der Weiterverarbeitung der Produktstapel 6 erreichbar.

Dem Transportsystem 12 ist u.a. eine Einschlagstation 16 zugeordnet, in der in einem geeigneten Folienspeicher, beispielsweise in Form von Rollen oder dergleichen die zur Bildung der Verpackungsbeutel vorgesehene Beutelfolie 10 vorgehalten ist. In der Einschlagstation 16 ist ein geeignet geformter Formschuh 18 vorgesehen, über den die Beutelfolie 10 um die entlang des Transportsystems 12 transportierten Produktstapel 6 gelegt wird, wobei die Papiertaschentücher in die Folie eingeschlagen werden. Der Formschuh 18 ist dabei derart ausgestaltet, dass die zugeführte Folie die durchlaufenden Produktstapel 6 vollständig umgibt, wobei an der Längskante des dabei entstehenden Beutelfolienschlauchs 4 eine Überlappung der entsprechenden Kantenbereiche der Folie eingestellt wird.

Die Einschlagstation 16 ist dabei mit geeigneten Mitteln zur örtlichen Synchronisierung der Produktstapel 6 mit der Beutelfolie 10 versehen. Dabei ist insbesondere vorgesehen, die Produktstapel 6 relativ zur Beutelfolie 10 korrekt zu positionieren, so dass die Lage der Produktstapel 6 relativ zu einer auf der Beutelfolie 10 aufgebrachten Bedruckung und/oder relativ zu einer darin eingebrachten Perforation vorgegebene Randbedingungen erfüllt. Damit kann beispielsweise sichergestellt sein, dass eine durch eine Perforation in der Beutelfolie 10 gebildete Zugriffsöffnung im Verpackungsbeutel auch geeignet relativ zum in den Verpackungsbeutel eingeschlagenen Produktstapel 6 positioniert ist.

Über das Transportsystem 12 sind die zu verpackenden Hygieneprodukte von der Einschlagstation 16 aus gemeinsam mit der sie umgebenden Beutelfolie 10 einer nachgelagerten ersten Schweißstation 20 zuführbar. Die erste Schweißstation 20 weist dabei in seitlicher Anordnung eine geeignet ausgelegte, insbesondere als Schweißspiegel ausgestaltete Heizeinrichtung 22 auf, über die der Folienschlauch 4 auf eine zum Schweißen ausreichend hohe Temperatur beheizbar ist. Infolge der Beheizung werden die überlappenden Randbereiche der Folie miteinander verschweißt, so dass in der ersten Schweißstation 20 der eigentliche Folienschlauch 4 entsteht. Der Folienschlauch 4 bildet somit eine durchgehende Umhüllung mehrerer aufeinander folgender Produktstapel 6 von Papiertaschentüchern.

Durch den Pfeil 24 ist die Transportrichtung der Produktstapel 6 der Papiertaschentücher auf dem Transportsystem 12 angedeutet. In dieser Transportrichtung gesehen nach der ersten Schweißstation 20 ist eine Vereinzelungseinheit 26 vorgesehen, über die der mit den Hygieneprodukten versehene Beutelfolienschlauch 4 in jeweils einen Produktstapel 6 beinhaltende Verpackungsbeutel vereinzelt wird. Diese Vereinzelung ermöglicht nachfolgend eine individualisierte Weiterverarbeitung jeweils einzelner in die Folie eingeschlagener Produktstapel 6. Die Vereinzelungseinheit 26 kann dabei beispielsweise geeignete Schneidmesser zum Durchtrennen des Beutelfolienschlauchs 4 zwischen zwei einander nachfolgenden Produktstapeln 6 aufweisen.

Nach der Vereinzelung der Produktstapel 6 mündet das Transportsystem 12 in eine Umlenkeinheit 28, in der der mit den Hygieneprodukten versehene Beutelfolienschlauch 4 relativ zur Transportrichtung um einen vorgegebenen Verdrehwinkel 32 gedreht wird. Der Verdrehwinkel 32 ist dabei derart gewählt, dass nach der Drehung ein seitlicher Zugriff auf die noch nicht verschweißten Seitenbereiche des Produktstapels 6 möglich ist. Dementsprechend entspricht der Verdrehwinkel 32 basierend auf der Grundfläche des Produktstapels 6 dem von zwei Seiten des Produktstapels 6 aufgespannten Winkel. Im Ausführungsbereich weist der Produktstapel 6 eine im Wesentlichen rechteckige Grundfläche auf, so dass ein Verdrehwinkel 32 von 90° gewählt ist.

Im Ausführungsbeispiel ist die Umlenkeinheit 28 dabei derart ausgestaltet, dass im Bereich der Umlenkeinheit 28 - wie durch den Pfeil 30 angedeutet - eine Änderung der Transportrichtung der Hygieneprodukte auf dem Transportsystem 12 um 90° erfolgt. Diese Änderung der Transportrichtung ist dabei vorgesehen, ohne dass eine Änderung der Orientierung der Produktstapel 6 mit dem sie umgebenden Beutelfolienschlauch 4 erfolgt, so dass sich relativ zur Transportrichtung gesehen die Orientierung der Produktstapel 6 in der Umlenkeinheit 28 um etwa 90° ändert.

Von der Umlenkeinheit 28 aus sind die in die Beutelfolie 10 eingeschlagenen Produktstapel 6 über das Transportsystem 12 einer nachgeschalteten zweiten Schweißstation 34 zuführbar. In dieser zweiten Schweißstation 34 erfolgt die Verschweißung der Stirnseiten der Verpackungsbeutel 2 für die Produktstapel 6, wobei auf Grund der Orientierung der Stapel 6 relativ zur Transportrichtung auch hier die Verschweißung von der Seite her erfolgen kann. Dazu umfasst die zweite Schweißstation 34 ebenfalls seitlich angeordnete Heizmittel 36, die beispielsweise als beidseitig zum Produktstrom angeordnete Heizspiegel ausgeführt sein können.

Anschließend mündet das Transportsystem 12 in eine der zweiten Schweißeinheit 34 nachgeschaltete Kühleinrichtung, in der eine Abkühlung der frisch verschweißten Verpackungsbeutel erfolgt.

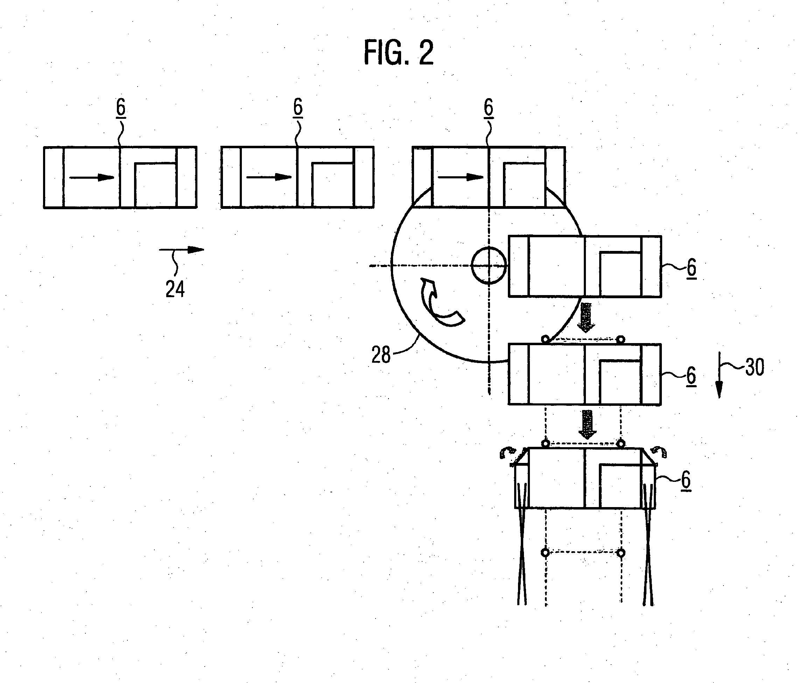

Zur besseren Darstellung ist die vorgesehene Änderung der Orientierung der Produktstapel 6 relativ zur Transportrichtung gesehen in FIG. 2 in vergrößerter Darstellung gezeigt. Wie dieser Darstellung entnehmbar ist, werden die Produktstapel 6 aus jeweils zehn Papiertaschentüchern im Bereich vor der Umlenkeinheit 28 entlang der durch den Pfeil 24 angedeuteten Transportrichtung in Längsrichtung, also in einer Orientierung mit ihrer Längsachse im Wesentlichen parallel zur Transportrichtung, transportiert. In der Umlenkeinheit 28 erfolgt, wie durch den Pfeil 30 symbolisiert, eine Richtungsumlenkung der Transportrichtung für die Papiertaschentücher, wobei die Übergabe innerhalb der Umlenkeinheit 28 derart erfolgt, dass die Orientierung der Produktstapel 6 unverändert bleibt.

Ausgangsseitig der Umlenkeinheit 28 werden die Produktstapel 6 somit mit ihrer Längsachse quer zur Transportrichtung oder mit einem Winkel von etwa 90° relativ zur Transportrichtung weiter transportiert.

Selbstverständlich könnte die nunmehr vorgesehene Änderung der Orientierung der Produktstapel 6 relativ zur Transportrichtung um etwa 90° alternativ auch dadurch erfolgen, dass unter Beibehaltung der eigentlichen Transportrichtung der eigentliche Produktstapel 6 um einen Winkel von etwa 90° um seine Hochachse gedreht wird.

Durch diese Orientierungsänderung des Produktstapels 6 relativ zur Transportrichtung ist in der Verpackungsanlage 1 gewährleistet, dass sämtliche Seitenbereiche des Verpackungsbeutels für die Papiertaschentücher von der Seite her und somit im Durchlaufbetrieb und ohne Systemstillstand verschweißt werden können.

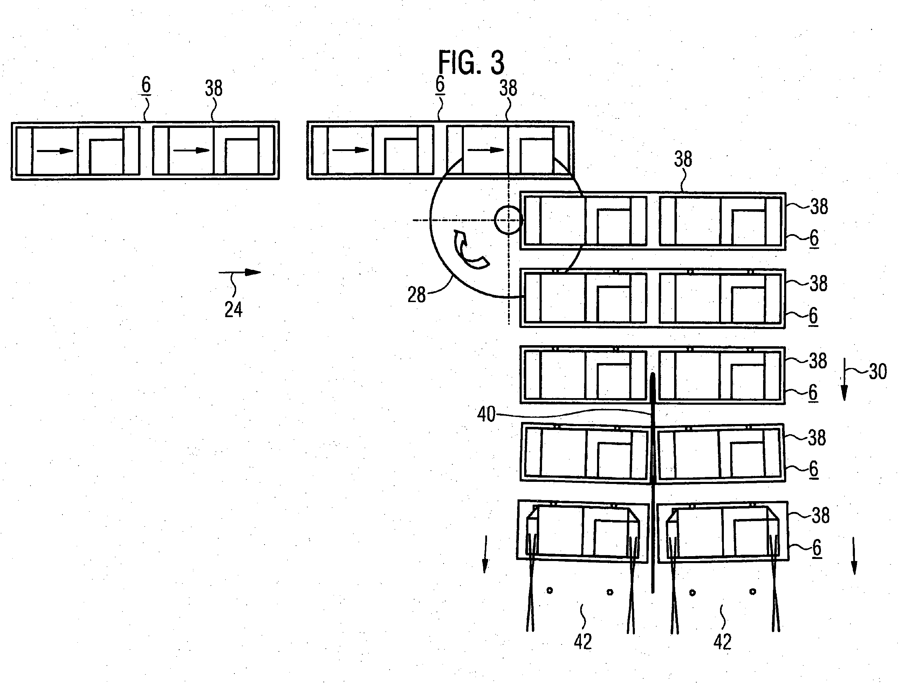

Eine alternative Ausführungsform des Transportsystems der Verpackungsanlage 1 ist in FIG. 3 gezeigt. In dieser Ausführungsform ist die Verpackungsanlage 1 gezielt dafür ausgelegt, dass nach dem ersten Schweißschritt und nach der Änderung der Orientierung der Produktstapel 6 relativ zur Transportrichtung eine mehrkanalige oder mehrsträngige Weiterverarbeitung der Produktstapel 6 in der Art einer Parallelbearbeitung gerade bei der Vornahme des zweiten Schweißschritts oder der Stirnschweißung möglich ist. Dazu wird im Ausführungsbeispiel gemäß FIG. 3 der mit den Produkten versehene, aus der ersten Schweißstation 20 austretende Beutelfolienschlauch 4 zunächst in Einheiten 38 vereinzelt, von denen jede jeweils mehrere - im gezeigten Ausführungsbeispiel zwei - Produktstapel 6 umfasst. Analog zum in FIG. 2 gezeigten Ausführungsbeispiel werden die Einheiten 38 anschließend in der Umlenkeinheit 28 derart umgelenkt, dass unter Beibehaltung ihrer Orientierung eine Änderung ihrer Transportrichtung, wie durch den Pfeil 30 symbolisiert, um etwa 90 ° erfolgt. Im Ausführungsbeispiel gemäß FIG. 3 werden somit die Einheiten 38 ausgangsseitig der Umlenkeinheit 28 mit ihrer Längsachse quer zur Transportrichtung oder mit einem Winkel von etwa 90 ° relativ zur Transportrichtung weitertransportiert.

Anschließend erfolgt in diesem Ausführungsbeispiel eine weitere Vereinzelung der Einheiten 38 derart, dass nunmehr die jeweiligen Produktstapel 6 voneinander getrennt oder separiert werden. Dazu ist im Ausführungsbeispiel gemäß FIG. 3 eine Schneideinheit 40, insbesondere ein geeignet positioniertes und dimensioniertes Schneidmesser, vorgesehen, das örtlich fixiert ist, und auf das die Einheiten 38 bei ihrem Weitertransport auflaufen. Durch dieses Auflaufen trennt die Schneideinheit 40 den Beutelfolienschlauch 4 im Bereich zwischen den Produktstapeln 6, so dass ausgangsseitig der Schneideinheit 40 einzelne, in dem Beutelfolienschlauch 4 eingeschlagenen Produktstapel 6 nebeneinander vorliegen. Die Produktstapel 6 können anschließend jeweils eigenständig einer nachfolgend angeordneten Weiterverarbeitung zugeführt werden. Dazu sind ausgangsseitig der Schneidstation 40 in der Art einer Parallelschaltung nebeneinander angeordnete Weiterverarbeitungsstränge 42 vorgesehen, von denen jeder jeweils eine geeignete zweite Schweißstation 34 zur Durchführung der Stirnschweißung aufweist. Der zweite Schweißschritt kann damit in der Art einer parallelen Verarbeitung mehrerer Produktstapel 6 gleichzeitig vorgenommen werden, so dass entsprechend erhöhte Durchsatzraten erreichbar sind.

### Bezugszeichenliste

- 1: Verpackungsanlage
- 4: (Beutel-)Folienschlauch
- 6: (Produkt-)Stapel
- 8: Zuführeinheit
- 10: Beutelfolie
- 12: Transportsystem
- 14: Zuführschienen
- 16: Einschlagstation
- 18: Formschuh
- 20: erste Schweißstation
- 22: Heizeinrichtung
- 24: Pfeil
- 26: Vereinzelungseinheit
- 28: Umlenkeinheit
- 30: Pfeil
- 32: Verdrehwinkel
- 34: zweite Schweißstation
- 36: Heizmittel
- 38: Einheiten
- 40: Schneideinheit
- 42: Weiterverarbeitungsstränge

## Patentansprüche

1. Verfahren zum Verpacken von Produkten, insbesondere von Hygieneprodukten, in Verpackungsbeutel, bei dem die zu verpackenden Produkte in einen Beutelfolienschlauch (4) eingeschlagen und dieser anschließend in einer Transportrichtung durch eine erste Schweißstation (20) und durch eine zweite Schweißstation (34) geführt wird, wobei der mit den Produkten versehene Beutelfolienschlauch (4) in einer Orientierung mit seiner Längsachse im Wesentlichen parallel zur Transportrichtung in einem ersten Schweißschritt durch die erste Schweißstation (20) hindurchgeführt und dabei seitlich verschweißt wird, bevor in einem zweiten Schweißschritt seine Stirnseiten verschweißt werden, und wobei zwischen den Schweißschritten der mit den Produkten versehene Beutelfolienschlauch (4) derart relativ zur Transportrichtung um einen dem von zwei aneinanderstoßenden Produktseiten aufgespannten Winkel entsprechenden Verdrehwinkel (32) gedreht wird, dass er nach der Drehung in einer Orientierung mit seiner Längsachse quer zur Transportrichtung weiter transportiert wird.

2. Verfahren nach Anspruch 1, bei dem der mit den Produkten versehene Beutelfolienschlauch (4) relativ zur Transportrichtung um etwa 90° gedreht wird.

3. Verfahren nach Anspruch 1 oder 2, bei dem der mit den Produkten versehene Beutelfolienschlauch (4) nach dem ersten Schweißschritt (20) in jeweils einen oder einige Produkte oder Produktstapel (6) beinhaltende Verpackungsbeutel vereinzelt wird.

4. Verfahren nach einem der Ansprüche 1 bis 3, bei dem die zu verpackenden Produkte der zur Bildung des Beutelfolienschlauchs (4) vorgesehenen Schlauchfolie synchronisiert zu einer in der Schlauchfolie angebrachten Perforierung zugeführt werden.

5. Verpackungsanlage (1) zum Verpacken von Produkten, insbesondere von Hygieneprodukten, in Verpackungsbeutel, insbesondere zur Durchführung des Verfahrens nach einem der Ansprüche 1 bis 4, mit einem Transportsystem (12), über das die zu verpackenden Produkte von einer Einschlagstation zunächst einer ersten Schweißstation (20) und von dieser aus einer zweiten Schweißstation (34) zuführbar sind, und mit einer in Transportrichtung der Produkte gesehen zwischen der ersten und der zweiten Schweißstation (20, 34) angeordneten Umlenkeinheit zur Drehung der Längsachse der mit den Produkten versehenen Verpackungsbeutel relativ zur Transportrichtung um einen vorgesehenen Verdrehwinkel (32).

6. Verpackungsanlage (1) nach Anspruch (5), mit einer in Transportrichtung gesehen nach der ersten Schweißstation (20) angeordneten Vereinzelungseinheit für die Verpackungsbeutel.

## Claims

1. Method for packaging products, in particular hygiene products, in packaging bags, in which the products to be packaged are wrapped in a bag film tube (4) and subsequently guided in a transport direction through a first welding station (20) and through a second welding station (34), wherein the bag film tube (4) provided with the products is guided, in an orientation in which its longitudinal axis is substantially parallel to the transport direction, through the first welding station (20) where its sides are welded together in a first welding step, prior to its end faces being welded together in a second welding step, and wherein, between the welding steps, the bag film tube (4) provided with the products is rotated relative to the transport direction through an angle of twist (32) corresponding to the angle enclosed by two adjacent product sides, such that said bag film tube is advanced after being rotated in an orientation with its longitudinal axis transverse to the transport direction.

2. Method according to claim 1, wherein the bag film tube (4) provided with the products is rotated through approximately 90° relative to the transport direction.

3. Method according to either claim 1 or claim 2, wherein the bag film tube (4) provided with the products is separated after the first welding step (20) into packaging bags each containing either one or a few products or a stack (6) of products.

4. Method according to any of claims 1 to 3, wherein the products to be packaged by the tube film provided for forming the bag film tube (4) are fed in a synchronised manner to a perforation applied to the tube film.

5. Packaging system (1) for packaging products, in particular hygiene products, in packaging bags, in particular for carrying out the method according any of claims 1 to 4, comprising a transport system (12) by means of which the products to be packaged can be fed from a wrapping station initially to a first welding station (20) and from there out of a second welding station (34), and comprising a deflection unit arranged between the first and second welding station (20, 34), when viewed in the transport direction, for rotating the longitudinal axis of the packaging bag provided with the products through an intended twist angle (32) relative to the transport direction.

6. Packaging system (1) according to claim 5, comprising a separating unit for the packaging bags arranged downstream of the first welding station (20) when viewed in the transport direction.

## Revendications

1. Procédé d'emballage de produits, en particulier de produits d'hygiène, dans des sachets d'emballage, dans lequel les produits à emballer sont enveloppés dans un film tubulaire pour sachets (4) celui-ci étant ensuite conduit dans une direction de transport à travers une première station de soudage (20) puis à travers une deuxième station de soudage (34), le film tubulaire pour sachets (4) muni des produits étant, orienté avec son axe longitudinal essentiellement parallèle à la direction de transport, amené vers la première étape de soudage, par laquelle, au passage de la première station de soudage (20) il est d'abord soudé latéralement, avant que ses côtés frontaux soient soudés lors de la deuxième étape de soudage et, entre les étapes de soudage, le film tubulaire pour sachets (4) muni des produits étant tourné, par rapport à la direction de transport, selon un angle de torsion (32) correspondant à l'angle défini par deux côtés de produit contigus de telle sorte que, après la rotation, il est transporté plus loin , orienté avec son axe longitudinal transversalement par rapport à la direction de transport.

2. Procédé selon la revendication 1, dans lequel le film tubulaire pour sachets (4) muni des produits est tourné d'environ 90° par rapport à la direction de transport.

3. Procédé selon la revendication 1 ou 2, dans lequel le film tubulaire pour sachets (4) muni des produits est, après la première étape de soudage (20), séparé en respectivement un ou plusieurs sachets d'emballage contenant des produits ou des piles de produits (6).

4. Procédé selon une des revendications 1 à 3, dans lequel les produits à emballer du film tubulaire prévu pour la formation du film tubulaire pour sachets (4) sont conduits de façon synchronisée à une perforation mise en place dans le film tubulaire.

5. Installation d'emballage (1) pour l'emballage de produits, en particulier de produits d'hygiène, dans des sachets d'emballage, en particulier pour la réalisation du procédé selon une des revendications 1 à 4, avec un système de transport (12) par le biais duquel les produits à emballer peuvent être conduits à partir d'une station d'enveloppement d'abord à une première station de soudage (20) et, de là, à une deuxième station de soudage (34), et avec une unité de renvoi disposée, vue dans la direction de transport, entre la première et la deuxième station de soudage (20, 34) pour la rotation, d'un angle de torsion prévu (32) par rapport à la direction de transport, de l'axe longitudinal des sachets d'emballage munis des produits.

6. Installation d'emballage (1) selon la revendication 5, avec une unité de séparation des sachets d'emballage disposée, vue dans la direction de transport, après la première station de soudage (20).
